# EUROPÄISCHE PATENTSCHRIFT

(11) **EP 0 372 174 B1**
(45) Veröffentlichungstag und Bekanntmachung des Hinweises auf die Patenterteilung: **07.12.1994**
(21) Anmeldenummer: 89117082.1
(22) Anmeldetag: 15.09.1989
(51) Int. Cl.: A61B 17/22

(54) **Lithotriptor**
Lithotriptor
Lithotripteur

(30) Priorität: 28.11.1988 DE 3840077
(43) Veröffentlichungstag der Anmeldung: 13.06.1990
(73) Patentinhaber: Richard Wolf GmbH, 75438 Knittlingen (DE)
(72) Erfinder: Wurster, Helmut, D-7519 Oberderdingen (DE); Krauss, Werner, D-7134 Knittlingen (DE)
(74) Vertreter: Wilcken, Hugo

(56) Entgegenhaltungen:
- EP-A- 0 260 550
- EP-A- 0 264 738
- FR-A- 2 591 467

## Beschreibung

Die Erfindung betrifft einen Lithotriptor gemäß den im Oberbegriff des Anspruches 1 aufgeführten Merkmalen.

Lithotripter mit den im Oberbegriff des Anspruches 1 angegebenen Merkmalen sind bekannt und in verschiedenen Ausgestaltungen auf dem Markt erhältlich. Beispielhaft sei hier ein Lithotriptor, wie er sich aus dem Prospekt "MFL 5000" von C. H. F. Mueller medizintechnische Systeme (Unternehmensbereich der Philips GmbH Hamburg) ergibt. Bei dem dort abgebildeten und beschriebenen Gerät erfolgt die bei jeder Lithotripsie erforderliche, der Zerstörung eines Steines mittels Ultraschall-Stoßwellen vorausgehende Ortung des Steines durch ein Röntgenortungssystem. Nach der erfolgten Ortung des zu zerstörenden Konkrementes werden dessen räumliche Koordinaten elektronisch ermittelt. Mittels einer komplizierten Elektronik und Mechanik wird danach der Ultraschall-Stoßwellenwandler so geführt, daß sein Fokus in den durch die ermittelten räumlichen Koordinaten festgelegten Bereich zu liegen kommt. Erst dann ist die Applikation von Ultraschall-Stoßwellen möglich und medizinisch sinnvoll. Ein wesentlicher Nachteil dieses Lithotriptors ist darin zu sehen, daß der Stoßwellenwandler nach der Ortung des zu zerstörenden Konkrementes mittels des Röntgenortungssystemes an den Patienten angekoppelt und mit seinem Fokus auf das Konkrement ausgerichtet werden muß. Wenn sich der Patient nach erfolgter Ortung bewegt oder die Ankopplung des Stoßwellenwandlers über das Koppelmedium an den Patientenkörper mit zu hohem Andruck erfolgt, kommt es vor, daß sich das Konkrement aus dem zuvor durch Röntgenortung ermittelten Punkt entfernt, so daß auch bei vorschriftsmäßiger Ausrichtung des Fokus auf den mittels Röntgenortung ermittelten Punkt, der Fokus des Wandlers nicht mit dem Konkrement übereinstimmt. Dies kann nicht nur die nachfolgende Behandlung mittels Stoßwellen verschlechtern oder wirkungslos machen, sondern möglicherweise auch zu Schäden im Gewebe fuhren. Im übrigen ist eine Kontrolle über den Grad der Konkrementzerstörung während der Behandlung nicht möglich.

Ein ähnlicher Lithotriptor ist in Biliary Lithotripsy, Adapted from the Proceedings of The First International Symposium on Biliary Lithotripsy, Boston, Massachusetts, 11.-13. Juli, 1988, Year Book Medical Publishers, Inc. des Jahres 1989, von Joseph T. Ferrucci, Michael Delius und H. Joachim Burhenne beschrieben, und zwar dort insbesondere in dem auf den Seiten 293 bis 299 abgedruckten Artikel "Diasonics: Therasonic Lithotripsy Treatment System" von J. Pell, A. Stein und K. W. Marich. Der dort beschriebene Lithotriptor entspricht im wesentlichen dem vorbeschriebenen, jedoch mit dem Unterschied, daß dem Stoßwellenwandler unmittelbar ein Ultraschallortungswandler zugeordnet ist, so daß auch nach erfolgter Röntgenortung eine gewisse Kontrolle der Behandlung über die Ultraschallortung möglich ist.

Schließlich ist es aus der Broschüre "Dornier Nierenlithotriptor nichtinvasive Nierensteinzerkleinerung" bekannt, dann, wenn die Röntgenortung erst nach der Ankopplung des Wandlers an den Patientenkörper erfolgt, das Koppelmedium in einem vorgegebenen Bereich um die Röntgenstrahlerachse herum mittels eines mit Gas aufblasbaren Ballons zu verdrängen, um eine bessere Bildqualität bei der Ortung zu erreichen. Das dort beschriebene System arbeitet mit zwei Röntgenortungssystemen und einer Funkenstrecke zur Stoßwellenerzeugung. Eine Ultraschallortung ist hier nicht vorgesehen. Abgesehen von der technisch sehr aufwendigen Steinortung mittels zwei Röntgenortungssystemen weist die dort beschriebene Anordnung den Nachteil auf, daß mögliche Hindernisse, die im Bereich zwischen Stoßwellenerzeuger und Konkrement liegen, durch die Röntgenortung nicht zuverlässig erfaßt werden können, da die Röntgenstrahlerachsen außerhalb der Laufstrecke der Stoßwellen liegen.

Ausgehend von dem einleitend beschriebenen Stand der Technik liegt der Erfindung die Aufgabe zugrunde, einen gattungsgemäßen Lithotriptor so auszubilden, daß die vorerwähnten Nachteile vermieden werden und ein Lithotriptor geschaffen wird, der einerseits technisch einfach im Aufbau und andererseits zuverlässig und einfach handhabbar ist.

Diese Aufgabe wird ausgehend von einem Lithotriptor, mit den im Oberbegriff des Anspruches 1 angegebenen Merkmalen durch die im kennzeichnenden Teil dieses Anspruches aufgeführten Merkmale gelöst.

Durch die erfindungsgemäße Maßnahme entfällt ein Nachführen bzw. Ausrichten des Stoßwellenwandlers auf die Achse des Röntgensystemes mittels einer komplizierten Steuerelektronik und Mechanik. Es ist lediglich das zu zerstörende Konkrement oder Gewebe während der Ortung in den Fokus zu bringen. Da das Röntgensystem schwenkbar angeordnet ist, kann eine dreidimensionale Röntgenortung erfolgen, wobei unabhängig von der Schwenkstellung des Röntgenortungssystemes stets sichergestellt ist, daß der Stoßwellenwandler korrekt in bezug auf das Röntgenortungssystem ausgerichtet ist. Durch die zentrale Anordnung des Röntgensystemes stimmt die Achse des Röntgensystemes stets mit der des Stoßwellenwandlers überein, was für eine exakte Ortung von besonderem Vorteil ist. Die dabei üblicherweise zu erwartende Verschlechterung des Röntgenbildes aufgrund des in der Röntgenstrecke befindlichen Koppelmediums, wird durch die Anordnung eines aufblasbaren Ballons im Koppelmedium verhindert. Wenn also das Röntgensystem zwecks Ortung des Konkrementes in Betrieb gesetzt wird, wird der Ballon mit Gas gefüllt, wodurch das flüssige Koppelmedium in einem hinreichend großen Bereich verdrängt wird. Dies gestattet den relativ ungedämpften Durchgang der Röntgenstrahlen. Nach erfolgter Ortung des zu zerstörenden Konkrementes oder des Gewebes im Köper des Patienten wird das Gas aus dem Ballon gesaugt, so daß die zur Zerstörung des Konkrementes oder des Gewebes ausgetrahlten Ultraschall-Stoßwellen im gesamten Ankoppelbereich durch die Flüssigkeit als Koppelmedium zum Körper des Patienten geleitet werden.

Weiterhin sieht die Erfindung vor, daß mindestens ein Ultraschall-Ortungswandler mit dem Stoßwellenwandler verbunden ist. Dieser Ultraschall-Ortungswandler kann beispielsweise ein B-Scanner sein, der mit dem Hauptwandler so verbunden ist, daß er dessen Fokusgebiet abscannen kann. Dadurch wird neben der Röntgenortung des Konkrementes oder des Gewebes die Ultraschallortung ermöglicht. Das Bedienungspersonal kann also wahlweise die eine oder andere Ortungsmethode anwenden, oder aber auch beide zugleich. In letzterem Falle, in dem der vorerwähnte Ballon mit Gas aufgeblasen worden ist, damit die Röntgenstrahlen vom Kopplungsmedium ungehindert durch den Körper des Patienten treten können, muß durch eine geeignete Formgebung des Ballons dafür gesorgt werden, daß die Ultraschallwellen des oder der Ultraschallortungswandler vom Ballon möglichst ungehindert das Zielgebiet erreichen können bzw. vom Zielgebiet reflektierte Wellen empfangen können.

Beide Ortungsmethoden können angewandt werden, ohne daß dabei der Patient verlagert werden muß. Darüberhinaus braucht das eine Ortungssystem nicht gegen das andere ausgetauscht zu werden. Dies hat den Vorteil, daß z. B. der Druck auf eine die erwähnte als Koppelmedium dienende Flüssigkeit einschließende, am Patientenkörper anliegende Membran konstant bleibt. Dadurch bedarf es keiner verlagerung des Patientenkörpers, welche eine Veränderung der Lage beispielsweise des zu zerstörenden Konkrementes bewirken könnte.

Im übrigen kann der Ultraschallwandler ein piezoelektrischer, ein magnetostriktiver Wandler oder ein Wirbelstromwandler sein. Aber auch ein Einsatz eines Wandlers mit einer Funkenentladungsstrecke ist durchaus möglich. Der Wandler kann kalottenförmig ausgebildet sein, so daß die von ihm ausgesendeten Ultraschallwellen von vornherein auf einen Fokus gerichtet sind. Aber auch der Einsatz eines planaren Ultraschallwandlers ist möglich. Zusätzlich zum Wandler muß dann allerdings zur Fokussierung eine geeignete Linse im Strahlengang der Ultraschallwellen vorgesehen sein, die die Ultraschallwellen auf einen Fokus hin bündeln.

Die Positionierung des Röntgensystems mit dem Stoßwellenwandler über bzw. am Körper des Patienten erfolgt gemäß einer vorteilhaften Weiterbildung mittels eines Antriebes. Dieser Antrieb ist in den Koordinaten X,Y und Z zur Positionierung des Fokus des Stoßwellenwandlers auf das zu zerstörende Konkrement oder Gewebe verfahrbar. Vorzugsweise ruht der Patient auf einem Behandlungstisch, dessen Platte eine Öffnung zum Durchtritt der Röntgenstrahlen und der Ultraschallwellen aufweist. Der betreffende Körperteil, in welchem sich das Konkrement oder das zu zerstörende Gewebe befindet, ist dabei im Bereich der erwähnten Öffnung positioniert.

Eine weitere Möglichkeit zur Positionierung des Fokus auf das Konkrement oder das zu zerstörende Gewebe ist dadurch gegeben, daß der Behandlungstisch selbst in den Koordinaten X,Y und Z verfahrbar ist, wobei das Röntgensystem mit dem Stoßwellenwandler in den Koordinaten X,Y und Z ortsfest, aber um den Fokus des Stoßwellenwandlers schwenkbar angeordnet ist.

Schließlich kann vorgesehen sein, das Röntgensystem zusammen mit dem Stoßwellenwandler an dem Rahmen anzuordnen, der mittels eines an einer vorzugsweise vertikalen Säule angreifenden Antriebs in vertikaler Richtung verfahrbar, um die Achse der Säule und um die Schwenkachse des Rahmens verschwenkbar ist. Auch hierdurch läßt sich die Positionierung des Röntgensystems und des Stoßwellenwandlers in einfacher Weise bewerkstelligen.

Die Erfindung wird anhand einiger Ausführungsbeispiele näher erläutert. Hierbei zeigt:
- Figur 1: ein erstes Ausführungsbeispiel des Lithotriptors in Anordnung an einem Behandlungstisch und in Verbindung mit einer Steuervorrichtung,
- Figur 2: eine vergrößerte Ansicht der in Figur 1 gezeigten Einheit aus einem Ultraschallwandler, einem Röntgenstrahler und zwei B-Scannern,
- Figur 3: die Schaltung der Einheit gemäß Figur 2 an eine Pneumatik,
- Figur 4: die Einheit gemäß Figur 2 mit halb aufgeblasenem Ballon,
- Figur 5: die Einheit gemäß Figur 2 mit eingezogenem Ballon,
- Figur 6: eine andere Ausführungsform der Einheit aus Röntgenstrahler, Ultraschallwandler und zwei B-Scannern unter Verwendung eines planaren Ultraschallwandlers und einer Linse,
- Figur 7: eine zweite Ausführungsform des Lithotriptors mit angedeutetem Behandlungstisch,
- Figur 8: eine dritte Ausführungsform des Lithotriptors in Anordnung an einen in X,Y und Z Richtung verfahrbaren Behandlungstisch, und
- Figur 9: eine weitere Ausführungsform des Lithotriptors.

Im Folgenden sind gleiche Teile mit den selben Bezugszeichen bezeichnet.

Die Einheit aus einem Ultraschallwandler 1 und dem Röntgenstrahler 2, welche weiter unten näher beschrieben wird, ist an einem freien Schenkel eines U-förmigen Rahmens 7 angeordnet. Am anderen Schenkel dieses Rahmens 7 ist dieser Einheit gegenüberliegend ein Bildverstärker 3 befestigt. Der U-förmige Rahmen 7 weist eine Schwenkachse 6 auf, die von einem auf einem verfahrbaren Ständer 10 sitzenden Antrieb 9 so angetrieben werden kann, daß der U-förmige Rahmen 7 um den Winkel α um die Schwenkachse 6 verschwenkt werden kann. Der Antrieb 9 vermag darüber hinaus, den U-förmigen Rahmen 7 in die angedeuteten X, Y und Z Richtungen zu verfahren.

Mit diesem Antrieb 9 ist also der Rahmen 7 und mit diesem das Röntgensystem 2,3 sowie der Ultraschallwandler 1 exakt positionierbar über einen Behandlungstisch 11, der von den Schenkeln des Rahmens 7 übergriffen wird.

Im Behandlungstisch 11 befindet sich eine Öffnung, in welcher der das zu zerstörende Konkrement oder das zu zerstörende Gewebe enthaltende Körperteil eines Patienten gelagert wird.

Der Röntgenstrahler 2 ist mit dem Wandler 1 fest verbunden und in dessen Zentrum so angeordnet, daß die Achse des Wandlers 1 und die Achse des Röntgenstrahlers 2 zusammenfallen.

Im gezeigten Ausführungsbeispiel sind darüber hinaus in der Kalotte des Wandlers 1 zwei Ultraschall-Ortungswandler 4 angeordnet, mittels derer neben der Röntgenortung des zu zerstörenden Konkrementes oder Gewebes eine Ultraschallortung vorgenommen werden kann.

Der Rahmen 7 weist eine Trennstelle 8 auf, um die der den Bildverstärker 3 tragende Schenkel des Rahmens 7 zur Seite schwenkbar ist. Dadurch wird eine Behinderung des behandelnden Arztes beseitigt, wenn das Gerät ausschließlich mit einer Ultraschallortung des Konkrementes oder Gewebes vorgenommen wird.

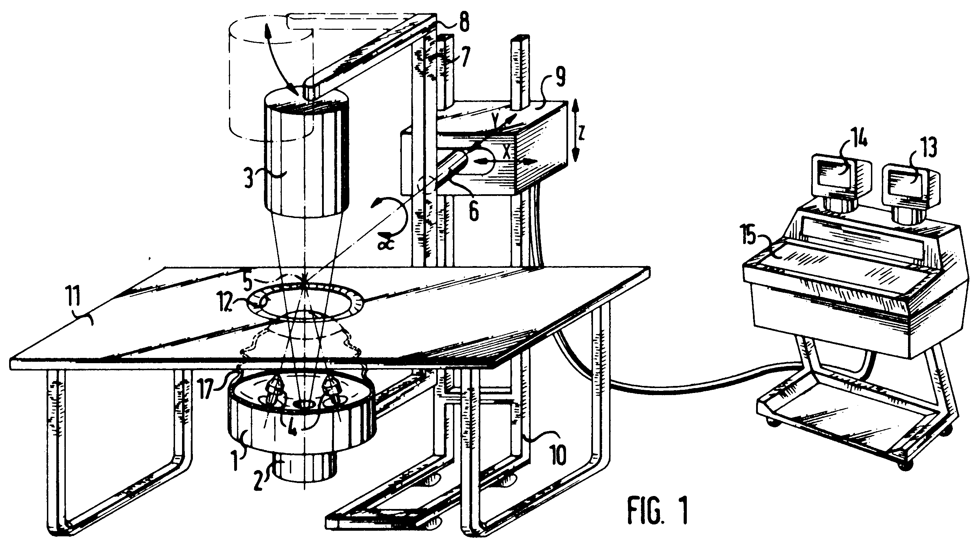

Im übrigen ist in Figur 1 eine sogenannte Untertischanordnung des Röntgenstrahlers 2 abgebildet, die eine nur geringe Belastung des Bedienungspersonals durch Röntgenstrahlen ermöglicht.

Der Lithotriptor ist mit einer Steuervorrichtung verbunden, die ein Bedienungspult 15 aufweist. Monitore 13 und 14 bilden die mittels de Röntgenortung bzw. Ultraschallortung gewonnenen Bilder ab.

Im übrigen ist das Iso-Zentrum für die Schwenkbewegung des Röntgensystems 2,3 bzw. dessen Rahmens der Fokus 5 des Ultraschallwandlers 1, wobei die gedachte Verlängerung der Schwenkachse 6 des Rahmens 7 bei jeder Verschwenkung um einen Winkel α den Fokus 5 schneidet.

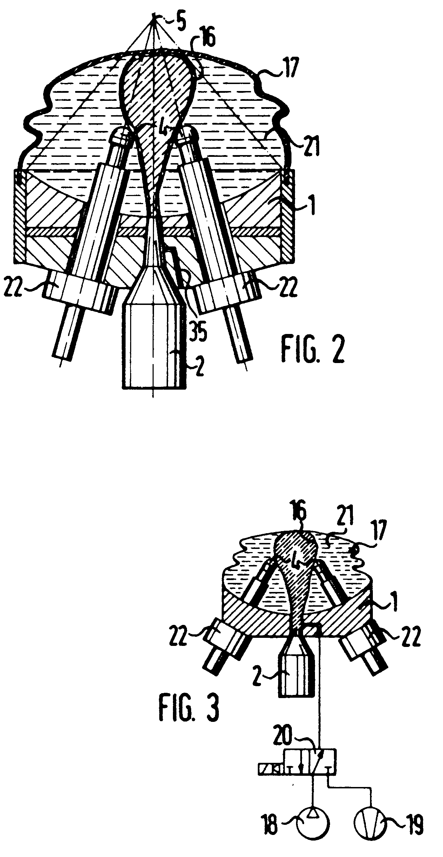

Figur 2 zeigt eine Ansicht der in Figur 1 gezeigten Einheit aus dem Ultraschallwandler 1, dem Röntgenstrahler 2 und zwei B-Scannern 4.

Der vorliegend als Kalotte ausgebildete Ultraschallwandler 1 weist eine zentraleöffnung auf, durch die der daran angeordnete Röntgenstrahler 2 seine Strahlung abgeben kann.

Zur besseren Ankoppelung des Ultraschallwandlers 1 an den Körper des Patienten ist die Kalotte des Wandlers 1 mit einer Flüssigkeit als Koppelmedium 21, bspw. Wasser, gefüllt und nach außen hin mit einer Membran 17 abgeschlossen.

In der werwähnten zentralen Öffnung im Ultraschallwandler 1 ist ein Ballon 16 vorgesehen, der, an seinem Einlaß bzw. Auslaß 35 gegenüber dem Koppelmedium 21 abgedichtet, mit Gas aufblasbar ist. Wenn dieser Ballon 16 aufgeblasen wird, wird das Koppelmedium 21 in einem bestimmten Bereich um die Achs des Röntgenstrahlers 2 herum verdrängt, so daß die Röntgenstrahlen praktisch ungedämpft in den Körper eines Patienten eintreten können.

Das Aufblasen des Ballons 16 mit Gas bzw. das Ablassen des Gases aus dem Ballon 16 erfolgt über eine Druckpumpe 18 bzw. eine Vakuumpumpe 19 (Figur 3), welche über ein Umschaltventil 20 und eine Verbindungsleitung mit dem Ballon 16 verbunden werden können.

Die Ultraschall-Ortungswandler 4 sind jeweils mit einem Scannergetriebe 22 verbunden, welches die Ortungswandler 4 längs deren Längsachse verfahren kann.

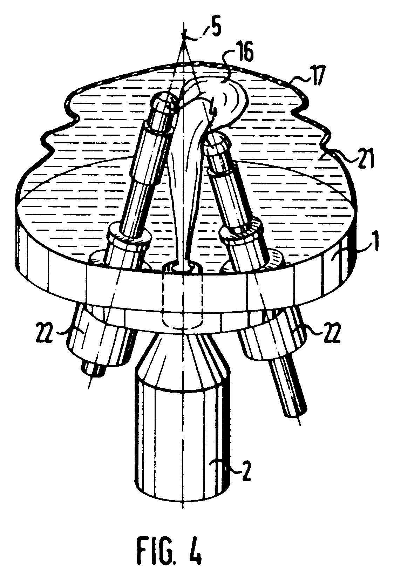

Figur 4 zeigt die Einheit aus dem Ultraschallwandler 1, dem Röntgenstrahler 2 und den Ortungswandlern 4, wobei hier der Ballon 16 nur etwa bis zur Hälfte gefüllt ist. Wie aus dieser Darstellung deutlich wird, ist es möglich, mit dem links im Bild dargetellten Ortungswandler 4 eine Ortung des zu zerstörenden Konkrementes oder Gewebes vorzunehmen, ohne den Ballon 16 vollständig entleeren zu müssen. Hierzu wird der betreffende Ortungswandler 4 mittels des Scannergetriebes 22 axial in Richtung des Fokus 5 verfahren, wobei der halbgefüllte Ballon 16 zur Seite gedrängt wird, so daß die vom Ortungswandler 4 ausgehenden Ultraschallwellen vom Ballon 16 ungehindert in den Körper des Patienten eindringen können.

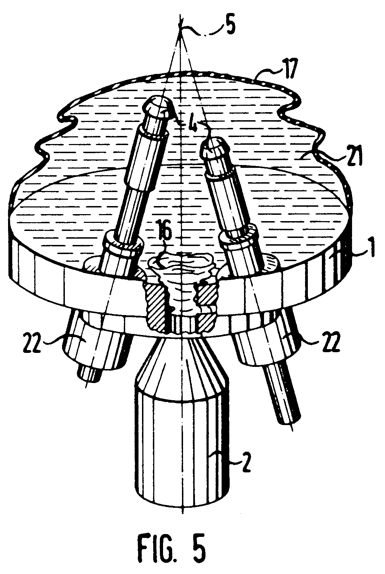

Figur 5 zeigt die erwähnte Einheit, wobei hier der Ballon völlig entleert ist. Diese Stellung hat der Ballon einzunehmen, wenn die vom Ultraschallwandler 1 erzeugten Ultraschall-Stoßwellen zur Zerstörung des Konkrementes oder Gewebes appliziert werden. Natürlich kann in dieser Stellung keine Röntgenortung mehr vorgenommen werden. Es ist aber klar, daß eine Ortung des Konkrementes durch eine Ultraschallortung mittels der Scanner 4 auch während der Applikation von Stoßwellen möglich ist, das heißt eine Realtime-Beobachtung des Zerstörungsvorganges kann vorgenommen werden.

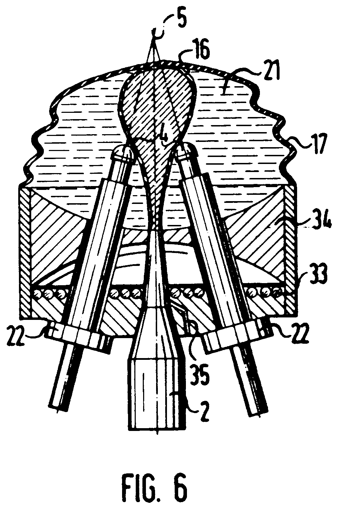

Figur 6 zeigt eine andere Ausführungsform der vorerwähnten Einheit, bei der anstelle eines kalottenförmigen Ultraschallwandlers ein planarer Ultraschallwandler 33 zur Anwendung kommt. Die Bündelung der Stoßwellen in dem Fokus 5 erfolgt durch eine akustische Linse 34.

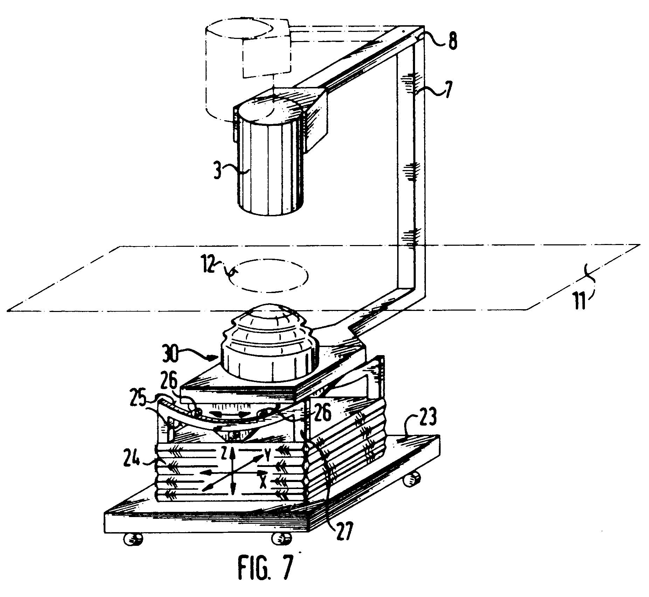

Figur 7 zeigt eine zweite Ausführungsform des erfindungsgemäßen Lithotriptors. Im Unterschied zu der ersten Ausführungsform gemäß Figur 1 erfolgt die Positionierung des Fokus 5 der nachfolgend mit dem Bezugszeichen 30 bezeichneten Einheit aus Ultraschallwandler 1, Röntgenstrahler 2 etc. auf das zu zerstörende Konkrement bzw. Gewebe durch einen auf einem Wagen 23 befestigten Untertisch-Antrieb 24, der den Rahmen 7 in X,Y und Z- Richtung verfahren kann.

Eine Schwenkbewegung des Rahmens 7 im weiter oben dargelegten Sinne wird durch ein motorisch betätigtes Abrollen von Rollen 26, die fest mit dem Rahmen 7 verbunden sind, auf am Antrieb 24 angebrachten Kufen 25 erzielt.

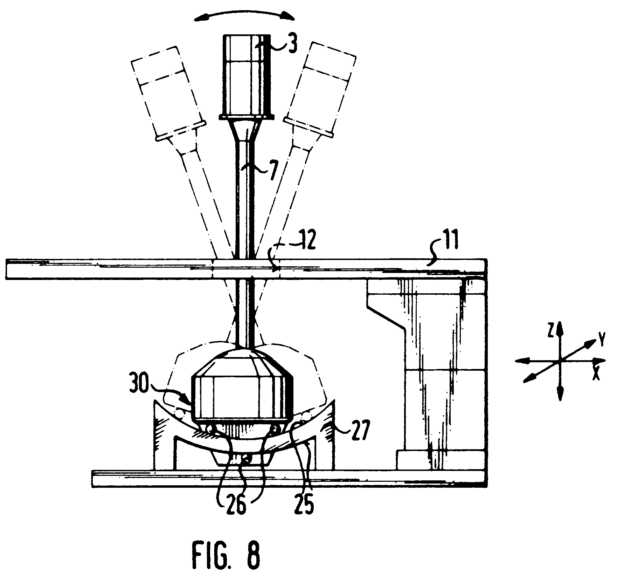

Figur 8 zeigt eine dritte Ausführungsform des Lithotriptors, bei dem die Positionierung des Fokus der Einheit 30 auf das zu zerstörende Konkrement oder Gewebe durch die Betätigung eines translatorisch in X,Y und Z-Richtung verfahrbaren Behandlungstisch 11, mit dem ein darauf liegender Patient in an sich bekannter Weise in den angegebenen Richtungen bewegt werden kann, erfolgt. Die für die Ortung des Konkrementes durchzuführende Schwenkbewegung erfolgt wie im Ausführungsbeispiel der Figur 7 durch ein Abrollen von Rollen 26 auf bogenförmigen Kufen 25.

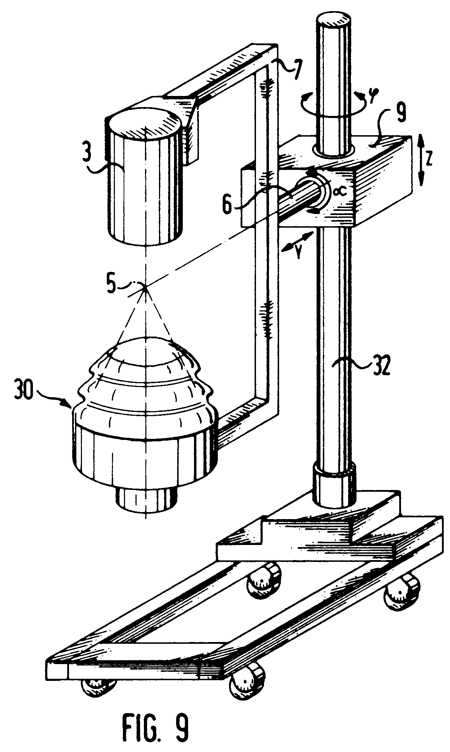

Figur 9 schließlich zeigt eine weitere Ausführungsform des Lithotriptors. Hier sind die Einheit 30 und der Bildverstärker 3 an einem Rahmen 7 angeordnet, der über die Schwenkachse 6 mittels eines an einer vertikalen Säule 32 angreifenden Antriebs 9 in vertikaler Richtung verfahrbar ist. Darüber hinaus gestattet der Antrieb 9 die Ausführung einer Bewegung in Richtung der Koordinate Y und eine Verschwenkbewegung um die Achse der Säule 32 um einen Winkel φ sowie ein Verschwenken um die Schwenkachse 6 des Rahmens 7 um einen Winkel α.

## Patentansprüche

1. Lithotriptor mit einem Wandler (1) zur Erzeugung von fokussierten Ultraschall-Stoßwellen, die über eine Flüssigkeit als Koppelmedium (21) auf den Körper des Patienten übertragen werden, wobei der Fokus (5) des Wandlers auf das jeweils zu zerstörende Konkrement oder Gewebe ausrichtbar ist, mit mindestens einem mit dem Wandler (1) verbundenen Ultraschall-Ortungswandler (4) und mit einem bildgebenden diagnostischen Röntgensystem (2, 3) zur Ortung des Konkrementes oder Gewebes, dessen Röntgenstrahler (2) und dessen dem Röntgenstrahler (2) mit Abstand gegenüberliegender Bildverstärker (3) an einem Rahmen (7) angeordnet sind, der zwecks Ortung in verschiedenen Bildebenen um eine Achse (6) verschwenkbar ist, dadurch gekennzeichnet,
- daß der Röntgenstrahler (2) mechanisch fest mit dem Wandler (1) verbunden und in dessen Zentrum angeordnet ist, derart, daß die Wandlerachse und die Röntgenstrahlerachse zusammenfallen,
- daß im Wandler (1) ein mit Gas aufblasbarer Ballon (16) vorgesehen ist, der im aufgeblasenen Zustand das Koppelmedium (21) in einem vorgegebenen Bereich um die Röntgenstrahlerachse herum verdrängt, und
- daß der Wandler (1) und das Röntgensystem (2, 3) um die gemeinsame Achse (6) schwenkbar sind, auf der der Fokus (5) des Wandlers (1) liegt.

2. Lithotriptor nach Anspruch 1, dadurch gekennzeichnet, daß das Röntgensystem (2, 3) zusammen mit dem Wandler (1) mittels eines Antriebes (9, 24) in den Koordinaten X, Y, Z zur Positionierung des Fokus (5) auf das zu zerstörende Konkrement oder Gewebe verfahrbar ist.

3. Lithotriptor nach Anspruch 1, dadurch gekennzeichnet, daß zur Positionierung des Fokus (5) auf das zu zerstörende Konkrement oder Gewebe ein in den Koordinaten X, Y, Z verfahrbarer Behandlungstisch (11) vorgesehen ist, auf dem der zu behandelnde Patient zum Liegen kommt und daß das Röntgensystem (2, 3) zusammen mit dem Wandler (1) in den Koordinaten X, Y, Z ortsfest und um den Fokus (5) verschwenkbar angeordnet ist.

4. Lithotriptor nach Anspruch 1, dadurch gekennzeichnet, daß das Röntgensystem (2, 3) zusammen mit dem Wandler (1) an dem Rahmen (7) angeordnet ist, der mittels eines an einer Säule (32) angreifenden Antriebes (9) in Richtung der Koordinaten Y und Z verfahrbar, um die Achse der Säule (32) um einen Winkel φ und um die Schwenkachse (6) des Rahmens (7) um einen Winkel α verschwenkbar ist.

## Claims

1. A lithotriptor with a transducer (1) for the production of focussed ultrasonic impulse waves, which are transmitted via a fluid as coupling medium (21) onto the body of the patient, in which the focus (5) of the transducer is able to be aligned to the concretion or tissue which is to be destroyed in each case, with at least one ultrasonic location transducer (4) connected with the transducer (1), and with a diagnostic X-ray system (2,3), producing an image, to locate the concretion or tissue, the X-ray emitter (2) of which and the image amplifier (3) of which, lying opposite the X-ray emitter (2) at a distance, are arranged on a frame (7), which for locating purposes is orientable in various image planes about an axis (6), characterised in that
- the X-ray emitter (2) is in mechanically fixed connection with the transducer (1) and is arranged in its centre, such that the transducer axis and the X-ray emitter axis coincide,
- in the transducer (1) a balloon (16) is provided, which is able to be inflated with gas, and which in inflated state displaces the coupling medium (21) in a prescribed region around the X-ray emitter axis, and
- the transducer (1) and the X-ray system (2,3) are pivotable about the common axis (6), on which the focus (5) of the transducer (1) lies.

2. A lithotriptor according to Claim 1, characterised in that the X-ray system (2,3) is movable together with the transducer (1) by means of a drive (9,24) in the coordinates X, Y, Z for the positioning of the focus (5) onto the concretion or tissue which is to be destroyed.

3. A lithotriptor according to Claim 1, characterised in that for the positioning of the focus (5) onto the concretion or tissue which is to be destroyed, a treatment table (11) is provided which is movable in the coordinates X, Y, Z, on which the patient who is to be treated comes to lie and that the X-ray system (2,3) together with the transducer (1) is arranged fixedly in the coordinates X, Y, Z and so as to be orientable about the focus (5).

4. A lithotriptor according to Claim 1, characterised in that the X-ray system (2,3) is arranged together with the transducer (1) on the frame (7), which is movable by means of a drive (9) engaging on a column (32) in the direction of the coordinates Y and Z, and is orientable about the axis of the column (32) through an angle φ and about the pivot axis (6) of the frame (7) through an angle α.

## Revendications

1. Lithotripteur comportant un transducteur (1) destiné à produire des ondes de chocs ultrasonores, qui sont transmises au corps du patient par l'intermédiaire d'un liquide faisant office de milieu de couplage (21), le foyer (5) du transducteur pouvant être orienté respectivement vers de la concrétion ou du tissu à détruire, l'appareil comportant au moins un transducteur de localisation (4) à ultrasons, relié au transducteur (1), et un système à rayons X de diagnostic (2, 3) fournissant une image et destiné à la localisation de la concrétion ou du tissu, dont l'émetteur de rayons X (2) et l'amplificateur d'image (3), opposé à distance à l'émetteur de rayons X (2), sont disposés sur un cadre (7), qui, dans le but de réaliser la localisation, peut être pivoté autour d'un axe (6), dans différents plans d'image, caractérisé
- en ce que l'émetteur de rayons X (2) est relié mécaniquement de manière fixe au transducteur (1), et est disposé au centre de celui-ci, de manière à ce que l'axe du transducteur et l'axe de l'émetteur de rayonnement soient confondus,
- en ce que, dans le transducteur (1), est prévu un ballon (16) pouvant être gonflé à l'aide de gaz, qui, à l'état gonflé, refoule le milieu de couplage (21), dans une zone prédéterminée, autour de l'axe de l'émetteur de rayons X, et
- en ce que le transducteur (1) et le système à rayons X (2, 3) peuvent être pivotés autour de l'axe commun (6), sur lequel se situe le foyer (5) du transducteur (1).

2. Lithotripteur selon la revendication 1, caractérisé en ce que le système à rayons X (2, 3) peut être déplacé, en commun avec le transducteur (1), au moyen d'un dispositif d'entraînement (9, 24), selon les coordonnées X, Y, Z, pour positionner le foyer (5) sur la concrétion ou le tissu à détruire.

3. Lithotripteur selon la revendication 1, caractérisé en ce que pour positionner le foyer (5) sur la concrétion ou le tissu à détruire, il est prévu une table de traitement (11) pouvant être déplacée selon les coordonnées X, Y, Z, sur laquelle est couché le patient, et en ce que le système à rayons X (2, 3), en commun avec le transducteur (1), est fixe en emplacement dans le système de coordonnées X, Y, Z et est agencé de manière à pouvoir être pivoté autour du foyer (5).

4. Lithotripteur selon la revendication 1, caractérisé en ce que le système à rayons X (2, 3), en commun avec le transducteur (1), est agencé au cadre (7), qui peut être déplacé dans la direction des coordonnées Y et Z au moyen d'un dispositif d'entraînement (9) venant en prise à une colonne (32), et peut être pivoté d'un angle φ autour de l'axe de la colonne (32), et d'un angle α autour de l'axe de pivotement (6) du cadre (7).
